Europäisches Patentamt

European Patent Office

Office européen des brevets

(11) Veröffentlichungsnummer: **0 348 808 B1**

(12) EUROPÄISCHE PATENTSCHRIFT

(45) Veröffentlichungstag der Patentschrift: **09.02.94**

(21) Anmeldenummer: **89111288.0**

(22) Anmeldetag: **21.06.89**

(51) Int. Cl.⁵: **A61K 9/16, A61K 9/52**

(54) Neue Arzneimittelformulierung sowie Verfahren zu deren Herstellung.

(30) Priorität: **30.06.88 DE 3822095**

(43) Veröffentlichungstag der Anmeldung:
**03.01.90 Patentblatt 90/01**

(45) Bekanntmachung des Hinweises auf die
Patenterteilung:
**09.02.94 Patentblatt 94/06**

(84) Benannte Vertragsstaaten:
**AT BE CH DE ES FR GB GR IT LI LU NL SE**

(56) Entgegenhaltungen:
EP-A- 0 202 051     EP-A- 0 255 002
EP-A- 0 263 083     WO-A-87/01588
DE-A- 3 233 764     GB-A- 2 134 785

(73) Patentinhaber: **Klinge Pharma GmbH
Berg-am-Laim-Strasse 129
D-81673 München(DE)**

(72) Erfinder: **Schneider, Gerhard, Dr.
Finkenstrasse 27
D-8011 Baldham(DE)**
Erfinder: **Stanislaus, Fritz, Dr.
Halserspitzstrasse 12
D-8000 München 80(DE)**
Erfinder: **Hofer, Josef Maximilian, Dr.
Bernauerstrasse 7
D-8018 Grafing(DE)**
Erfinder: **Heese, Gerd-Ulfert
Heilwigstrasse 2
D-8000 München 82(DE)**
Erfinder: **Huber, Hans-Joachim, Dipl.-Biologe
Ramoltstrasse 28
D-8000 München 83(DE)**

(74) Vertreter: **Kolb, Helga, Dr. Dipl.-Chem. et al
Hoffmann, Eitle & Partner,
Patentanwälte,
Postfach 81 04 20
D-81904 München (DE)**

EP 0 348 808 B1

**Beschreibung**

Die Erfindung betrifft eine neue Diclofenac-Natrium enthaltende Arzneimittelformulierung mit gesteuerter Wirkstoff-Freisetzung.

Für verschiedene Applikationen sind Arzneimittel erwünscht, die eine langanhaltende Bioverfügbarkeit gewährleisten. So verfügen z.B. auf dem Gebiet der nicht-steroiden Antirheumatika mit Ausnahme derjenigen aus der Gruppe der Oxicame die meisten über Plasmaeliminations-Halbwertzeiten von weniger als ca. 10 h (H. Fenner "Pharmakokinetik nicht-steroider Antirheumatika", Tempo Medical, Heft 12 A/82). Diese kurzen Plasmaeliminations-Halbwertzeiten sind wünschenswert, um das Risiko von Interaktionen mit anderen Arzneimitteln, die z.B. bei Anwendung von nicht-steroiden Antirheumatika mit längeren Plasmaeliminations-Halbwertzeiten verstärkt auftreten, zu vermindern. Dabei ist zu berücksichtigen, dass nicht-steroide Antirheumatika mehr oder weniger stark einer Bindung an Plasmaproteine unterliegen. Mit der Stärke dieser Bindung ist die Tendenz der Verdrängung anderer Wirkstoffe aus ihrer Bindung mit Plasmaproteinen verbunden. Hohe Bindungsaffinität korreliert somit mit einer hohen Interaktionsgefahr. Eine deutliche Herabsetzung der Plasmaproteinbindung als Folge der Biotransformation oder einer gezielten Strukturabwandlung hat eine Erhöhung des freien (=wirksamen) Anteils an Wirkstoff im Plasma zur Folge, der entsprechend rascher renal eliminiert wird. Dadurch kann eine tägliche Mehrfachapplikation erforderlich werden.

GB-A-2134785 betrifft eine langsam freisetzende Arzneimittelform für den Wirkstoff Pinacidil. Diese Arzneimittelform setzt sich aus zwei Typen von Pellets zusammen, wobei ein Teil eine schnelle Freisetzung im Magen, der andere Teil eine langsame Freisetzung im Darm erfährt.

EP-A-0202051 offenbart allgemein Arzneimittelformen, die sich aus bis zu drei unterschiedlichen Pellet-Typen zusammensetzen können, um auf diese Weise den Wirkstoff zu verschiedenen Zeiten im Verdauungstrakt des Patienten freizusetzen. Die vorstehende Druckschrift faßt sich nicht mit einem Magenschleimhaut-reizenden Wirkstoff, so daß sie auch nicht das Problem angeht, den Wirkstoff ungelöst durch den Magentrakt zu bringen, andererseits aber bereits kurzzeitig nach der Verabreichung einer ausreichenden Konzentration des Wirkstoffes im Plasmaspiegel sicherstellt und diesen Wert langfristig aufrechterhält.

Bei allen diesen Überlegungen ist zu berücksichtigen, dass ein grosser Anteil der Patienten, die eine Langzeitbehandlung mit nicht-steroiden Antirheumatika benötigen, im höheren Lebensalter sind. Bei Patienten dieser Altersgruppe ist die Wahrscheinlichkeit gross, dass ausser der "antirheumatischen" Therapie weitere medikamentöse Behandlungen indiziert sind. Die häufig zusammen mit Antirheumatika verabreichten Wirkstoffe gehören wegen ihrer pharmakokinetischen Eigenschaften in die Kategorie der potentiell interagierenden Substanzen und haben in vielen Fällen eine geringe therapeutische Breite. Hervorzuheben sind hier z.B. Wirkstoffe aus der Gruppe der Antikoagulantien und oralen Antidiabetika, die ebenfalls einer hohen Bindung an Plasmaproteine unterliegen.

Um z.B. die bei Verwendung von nicht-steroiden Antirheumatika mit kürzerer Plasmaeliminations-Halbwertzeit erforderliche tägliche Mehrfachapplikation auf eine maximal 2- bis 3-malige Arzneimittelgabe zu reduzieren, ist die Konzeption von Retardarzneiformen erforderlich. Damit soll der schlechten "patient-compliance", die unretardierte Antirheumatika durch die oftmalige tägliche Einnahme haben, begegnet worden (Vortrag Prof. Nürnberg/APV-Kurs über Antirheumatika, Nürnberg 19./20.11.1983). Dabei sind dem Fachmann aber durch die relativ kurzen Plasmaeliminations-Halbwertszeiten Grenzen auferlegt. So schreibt z.B. Fenner in der vorstehend erwähnten Veröffentlichung in Tempo Medical: "Die Verabreichung von nicht-steroiden Antirheumatika in Formulierungen mit verzögerter Wirkstoff-Freisetzung, z.B. Retard-Formulierungen von Diclofenac und Indometacin, ist nur bedingt geeignet, den aus den kurzen Plasmaeliminations-Halbwertszeiten dieser Substanzen resultierenden Plasmaspiegelverlauf im gewünschten Masse zu beeinflussen. Die Retardierung führt zu einer deutlichen Abflachung der Plasmaspiegelkurve; länger als ca. 8 bis 10 Stunden kann jedoch durch galenische Massnahmen keine nennenswerte Steuerung des Plasmaspiegels erfolgen."

Anhand der Darlegung der Diclofenac-Plasmaspiegelverläufe nach Verabreichung von einem Retard-Dragee zu 100 mg bei gesunden Probanden (n = 8) geht aus der Mittelwertkurve hervor, dass im Zeitraum zwischen 8 und 10 Stunden der Diclofenac-Plasmaspiegel auf Werte unterhalb von 100 ng/ml absinkt.

Die Schwelle von 100 ng/ml dürfte als diejenige Minimalkonzentration angesehen werden, deren Überschreitung zum Erhalt eines therapeutischen Effektes erforderlich ist.

Dem Fachmann ist nun besonders die Problematik bekannt, die darin liegt, Diclofenac-Plasmaspiegel derart lange aufrechtzuerhalten, dass einerseits die Wirkdauer über die Nachtruhe gegeben ist und Morgensteifigkeit verhindert wird, andererseits aber eine rasche Wirkung nach der Einnahme des Arzneimittels eintritt.

Hierzu geht aus einer Arzneimittelpackungsbeilage hervor, die Nachtschmerzen und Morgensteifigkeit durch kombinierte Einnahme eines magensaftresistenten Dragees mit der Verabreichung eines Zäpfchens oder Retard-Dragees vor dem Einschlafen zu vermindern.

Die kombinierte Einnahme mehrerer Arzneiformen ist jedoch stets mit der Gefahr einer mangelnden "patient-compliance" verbunden. So kann der Patient insbesondere bei den festen Arzneiformen die Einnahme der zweiten Arzneiform vergessen oder aber anstelle eines magensaftresistenten Dragees und eines Retarddragees zwei magensaftresistente oder zwei Retard-Arzneiformen einnehmen. Die Folge davon kann der Eintritt erhöhter Nebenwirkungen bzw. das Ausbleiben einer raschen Schmerzlinderung sein.

Somit würde es einen grossen Fortschritt darstellen, wenn es gelänge, die für einen therapeutischen Effekt erforderlichen Plasmaspiegel von z.B. ca. 100 ng Diclofenac/ml nach Einnahme einer einzigen Arzneiform schnell zu erreichen und lange Zeit über dieser Minimalkonzentration aufrechtzuerhalten.

In der JP 61/044811 wird eine Granulatmischung beschrieben, die sich aus einem initial-freisetzenden Anteil und einem verzögert-freisetzenden Anteil zusammensetzt. Dabei wird im initial-freisetzenden Anteil der Wirkstoff bereits im Magen freigesetzt. Im verzögert-freisetzenden Anteil ist das Granulat mit einer magensaftresistenten Membran umhüllt; in einer alternativen Ausführungsform wird der Wirkstoff mit dem magensaftresistenten Material vermischt (durchgeknetet).

Die Kombination eines initial-freisetzenden Granulatgemisches mit verzögert-freisetzendem Wirkstoffanteil wird auch in EP-A-255002 beansprucht.

Nicht-steroide Antirheumatika wirken über eine Hemmung der Prostaglandin-Biosynthese. Damit verbunden ist ein gewisses Potential der Magenschleimhaut-Reizung/Schädigung durch Verminderung auch derjenigen Prostaglandine, die einen Schleimhaut-protektiven Charakter haben (Drugs 32 (Suppl. 4): 27 [1986]). Andererseits haben die meisten nicht-steroiden Antirheumatika auch ein direktes, die Schleimhaut reizendes/schädigendes Potential. So konnte für Fenoprofen-Calcium gezeigt werden, dass eine magensaftresistent umhüllte Arzneiform weniger Nebenwirkungen verursacht als die nicht-umhüllte Arzneiform (Clin. Pharmacol. Ther., 42: 28 [1987]).

Aus diesem Grunde wurde der in den beiden oben genannten Schriften beschriebene Weg nicht als der sinnvollerweise gangbare angesehen, insbesondere auch, da aus EP-A-255002 nicht erkennbar ist, ob die für den therapeutischen Effekt erforderlichen Plasmaspiegel schnell nach Einnahme der Arzneiform erreicht werden, da der erste Zeitpunkt der Blutentnahme erst bei 2 Stunden liegt.

In DE-OS 34 31 861 wird eine Pellet-Zubereitung beschrieben, die u.a. einen Wirkstoff und ein Beschwerungsmittel enthält und mit einer magensaftresistenten Membran umhüllt ist. Die Pellets können in Hartgelatinekapseln oder in Form von Tabletten mit einem Bestandteil kombiniert werden, aus dem der Wirkstoff initial freigesetzt wird. Die gezeigten Blutspiegelkurven wurden an 2 bzw. 4 Probanden ermittelt, was für eine Beweisführung unzureichend ist.

Dem Vorgehen liegt der Wunsch einer Verlängerung der Verweildauer der Arzneiform im Magen zugrunde. Ob dies generell damit erreichbar ist, muss im Hinblick auf die Literatur, aus der kein Einfluss der Dichte der Arzneiform Pellet auf die Magenverweilzeit hervorgeht, bezweifelt werden (New Engl. J. Med., 304: 1365 [1981]).

Aus diesem Grunde wurde dieser Weg zum Erreichen des gewünschten Effektes nicht beschritten, sondern Untersuchungen zum Auffinden einer anderen Lösung durchgeführt.

Aufgabe der vorliegenden Erfindung ist es somit, einer mangelnden "patient-compliance" entgegenzuwirken, und ein Kombinationspräparat, welches in einer Arzneiform vorliegt, zur Verfügung zu stellen. Im weiteren soll die Aufgabe gelöst werden, dass bei der Einnahme des Kombinationspräparates möglichst sofort eine Schmerzlinderung eintritt, d.h. sich die Wirkung des Arzneimittels rasch entfaltet und diese über einen möglichst langen Zeitraum erhalten bleibt. Es soll verhindert werden, dass ein wesentlicher Teil des Wirkstoffes bereits im Magen freigesetzt wird; andererseits soll ein Teil des Wirkstoffes sehr schnell im oberen Teil des Dünndarms freigesetzt werden, um einerseits Schädigungen der Magenschleimhaut zu vermeiden, zum anderen aber eine rasche Schmerzlinderung zu erzielen.

Die vorstehende Aufgabe wird gemäss der Erfindung durch ein Diclofenac-Natrium enthaltendes Arzneimittel mit gesteuerter Wirkstoff-Freisetzung gelöst, welches dadurch gekennzeichnet ist, daß der Wirkstoff zu einem Teil in retardiert freisetzender und zum restlichen Teil in einer magensaftresistenten Form vorliegt, wobei der genannte Wirkstoff in oder auf sphärischen Pellets fein verteilt ist und ein Teil der Pellets mit einer für den Wirkstoff retardiert durchlässigen Diffusionsmembran und der restliche Teil der Pellets mit einer magensaftresistenten Membran umhüllt ist, und die genannten Pellets in einer Gelatinekapsel eingeschlossen sind.

Insbesondere soll gemäss der Erfindung der Wirkstoff in oder auf sphärischen Granulaten (Pellets) fein verteilt in amorpher, mikrokristalliner oder molekulardisperser Form vorliegen. Die Bezeichnung "magensaftresistent" bedeutet gemäss der Erfindung, dass dieser Bestandteil der Arzneiform gegenüber Magensaft beständig ist, während im Darmsaft eine rasche Freisetzung des Wirkstoffes erfolgt. Als magensaftresistent wird eine Arzneiform definiert, aus der gemäss den Bedingungen, wie sie in der Ergänzung zur US Pharmacopeia (USP XXI-NF XVI, Supplement No. 5) sowie im Vorschlag der Europäischen Arzneibuch-Kommission vom 17.7.1987 beschrieben sind, der Wirkstoff beim Säuretest (0,1 N HCl, 2 h, 37°C) zu nicht mehr als 10 % freigesetzt wird, während gemäss dem USP XXI-NF XVI, Supplement No. 5, bei pH 6,8 nach 45 Minuten der Wirkstoff zu mehr als 75% freigesetzt sein muss, um den Anforderungen des Arzneibuches zu entsprechen. Gemäss der Erfindung können alle möglichen Arzneiformen bzw. Teile davon magensaftresistent gestaltet sein, wie Kapseln, Tabletten und Dragees, insbesondere jedoch Pellets (Granulate) und Pulver. Gemäss der Erfindung sind Pellets besonders bevorzugt.

Als "retardiert freisetzend" bezeichnet man gemäss der Erfindung den Anteil der Arzneiform, die je nach Wahl der chemischen Zusammensetzung und Dicke der Membranhülle den Wirkstoff in der Hauptsache im Dünndarm in therapeutisch erforderlichen Mengen über einen längeren Zeitraum (6 bis 8 h) freisetzt. Es ist wünschenswert, dass im Magensaft keine nennenswerte Wirkstoff-Freisetzung erfolgt, da der Wirkstoff im wesentlichen im oberen Dünndarm absorbiert wird.

Es ist bevorzugt, dass das Arzneimittel gemäss der Erfindung in einer Kapsel, besonders bevorzugt einer Hartgelatinekapsel, eingeschlossen ist.

Als bevorzugte Arzneiform, die beide Anforderungen erfüllen kann, bietet sich z.B. eine Kapsel, bevorzugt eine Hartgelatinekapsel an, die einzelne Arzneibestandteile, wie z.B. sphärische Granulate (Pellets) enthält. Ein Teil der Pellets kann dabei mit einer magensaftresistenten Membran, ein anderer Teil mit einer für den Wirkstoff retardiert durchlässigen Diffusionsmembran umhüllt sein. Derjenige Anteil, der mit der magensaftresistenten Membran umhüllt ist, stellt ein rasches Erreichen des therapeutischen Effektes sicher, während derjenige Anteil, der mit der durchlässigen Diffusionsmembran umhüllt ist, durch langsame Wirkstoff-Freisetzung das lange Anhalten der Wirkung steuert.

Eine solche Arzneiform mit vielen einzelnen Komponenten (sog. "multiple-unit"-Arzneiform") hat evtl. gegenüber einer Ein-Komponenten-Arzneiform (sog. "singel-unit"-Arzneiform), die im vorliegenden Fall z.B. aus einem Retarddragee und einem magensaftresistenden Dragee, beide eingelegt in eine Hartgelatinekapsel, bestehen könnte, zusätzliche Vorteile: So werden im ersten Fall nach Auflösung der Hartgelatinekapsel im Magen, die ca. 5 Minuten nach Einnahme der Arzneiform beendet ist, mehrere 100 einzelne Arzneistoffträger freigesetzt, im letzteren Fall jedoch nur zwei. Da diese den Magen nur innerhalb der regulären Entleerungszyklen verlassen können und der bevorzugte Absorptionsort von z.B. nicht-steroiden Antirheumatika der obere Dünndarmbereich ist, sind einige wenige grosse Arzneistoffträger (Durchmesser > 2,0 mm) in ihrem Absorptions- und damit Wirkverhalten stärker abhängig vom Einfluss gleichzeitig aufgenommener Nahrung (Zeitpunkt der Nahrungsaufnahme, Menge und Zusammensetzung der Nahrung) oder gleichzeitig aufgenommener anderer Pharmaka (z.B. trizyklischer Antidepressiva, die den Zyklus der Magenentleerung verlangsamen können). Siehe hierzu auch Wegener, Schaffstein, Börsch in Medizinische Klinik, Nr. 10, 1988, Seiten 335-341, und Frömming in Der Internist, Nr. 27, 1986, Seiten 32-39.

Dem Fachmann ist auch bekannt, dass "multiple-unit"-Arzneiformen im direkten Vergleich zu den "single-unit"-Arzneiformen oftmals wesentlich rascher vom Magen aus in den Bereich des oberen Dünndarms entleert werden (z.B. Bechgaard, Acta Pharmaceutica Technologica 28, Nr. 2, 1982, Seiten 149-157).

Die Schwierigkeit bei der Formulierung einer derartigen Arzneiform liegt überwiegend in dem Anteil der Pellets, der mit der magensaftresistenten Membran umhüllt ist.

Aufbauend auf der Tatsache, dass orale Arzneiformen mit einem hohen Gehalt an alkalisch reagierenden Inhaltsstoffen nicht sicher magensaftresistent sind, sondern bereits im sauer reagierenden Magensaft zerfallen, wodurch der therapeutische Effekt des Arzneimittels vernichtet wird, werden gemäss der Erfindung besondere Massnahmen ergriffen. Vor dem Aufbringen der magensaftresistenten Lackschicht auf die Dosierungseinheit wird gemäss DE-OS 32 33 764 eine saure Isolierschicht aufgetragen, die als Hauptbestandteil wasserlösliche Celluloseether, vorzugsweise Hydroxypropylmethylcellulose, sowie ausserdem 15 bis 30 Gew.-% einer wasserlöslichen, festen, kristallinen, nichtflüchtigen, pharmakologisch akzeptablen ein- oder mehrbasischen organischen, vorzugsweise langkettigen Säure und 5 bis 15 Gew.-% eines wasserlöslichen Weichmachers, jeweils bezogen auf die Menge der Celluloseether, enthält. Durch diese Massnahme wird verhindert, dass durch Spuren von Feuchtigkeit, die entweder bereits bei der Filmbeschichtung, bei der unsachgemässen Lagerung oder im sauer-wässrigen Milieu des Magensaftes in die Dosierungseinheit eindringt, die alkalisch reagierenden Inhaltsstoffe die freien Carboxylgruppen des polymeren Filmbildners ionisieren, so dass der Filmbildner wasserlöslich wird. Weiteres Eindringen von Wasser kann dann grosse Mengen der alkalischen Inhaltsstoffe lösen, so dass der gesamte Filmüberzug aufgelöst wird.

Der Vorteil dieses Verfahrens wird beispielhaft für die Arzneiformen Weichgelatinekapseln, Hartgelatinekapseln und Filmtabletten dargelegt.

Zur magensaftresistenten Umhüllung können beispielsweise folgende pharmakologisch unbedenkliche Polymere verwendet werden: Copolymerisate mit anionischem Charakter auf Basis von Methacrylsäure und Methacrylsäuremethylester mit verschiedenem Verhältnis der freien Carboxylgruppen zu den Estern und einem mittleren Molgewicht von 135.000. Typische Vertreter dieser Stoffklassen sind z.B. die von der Firma Röhm-Pharma vertriebenen Acrylharzsubstanzen Eudragit[(R)]L und Eudragit[(R)]S bzw. als wässrige Dispersion Eudragit[(R)]L 30 D. Eine weitere Gruppe entstammt den Celluloseethern, die mit Phthalsäureanhydrid verestert sind. Handelsprodukte der Hydroxypropylmethylcellulosephthalate (HPMCP) tragen die Bezeichnung HP[(R)] 50 oder HP[(R)]55 und werden z.B. von der Firma Shin-Etsu Chemical und Co. hergestellt. HP[(R)]50 und HP[(R)]55 unterscheiden sich dabei in ihrem Gehalt an Methoxy-, Hydroxypropoxy- und Carboxybenzol-Gruppen (zur weiteren Information über die verwendeten Polymere s. z.B. Produktinformationen der Firmen Röhm-Pharma oder Shin-Etsu Chemcial bzw. Handbook of Pharmaceutical Excipients, USA 1986).

Die den Wirkstoff retardiert freisetzenden Pellets werden erfindungsgemäss mit einer im Magendarmtrakt unlöslichen, für den Wirkstoff retardiert durchlässigen Membran umhüllt.

Zur Herstellung der Membran können beispielsweise folgende pharmakologisch unbedenkliche Polymere verwendet werden:

Acrylsäureester, Methacrylsäureester, Copolymere der Acryl- und Methacrylsäureester, Vinylacetate, modifizierte Cellulose-Derivate etc.

Besonders geeignete Polymere zur Herstellung der Membran sind unter anderem Copolymere von Methacrylsäure und Methacrylsäureester mit variabel einstellbarem Gehalt an quaternären Ammoniumgruppen, die das Ausmass der Hydrophilität und somit auch der Permeabilität der Polymeren bestimmen.

Typische Vertreter dieser Stoffklasse sind z.B. die von der Firma Röhm-Pharma vertriebenen Acrylharze Eudragit[(R)]RL und Eudragit [(R)]RS. Diese Polymere weisen ein Ammoniumgruppenverhältnis von ca. 1:20 (Eudragit[(R)]RL und ca. 1:40 (Eudragit[(R)]RS) - molares Verhältnis der Ammoniumgruppen zu neutralen Acrylsäureestern - auf. Die Permeabilität der Eudragit[(R)]RL/RS Membran kann durch das Mischungsverhältnis der Komponenten beliebig eingestellt werden. Das für eine gewünschte Freisetzung erforderliche Mischungsverhältnis ist für die einzelnen Wirkstubstanzen in bekannter Weise zu ermitteln; es liegt üblicherweise in den Grenzen von 20:80 Gew.-% bis 80:20 Gew.-% Eudragit[(R)]RL : Eudragit[(R)]RS. Die Permeabilität der Diffusionsmembran kann zusätzlich noch durch Zugabe von Weichmachersubstanzen (Phthalsäuredibutylester, Triacetin etc.) und eventuell weiterer Hilfsstoffe, wie Talkum oder Magnesiumstearat, als Trenn- und Glättungsmittel beeinflusst werden.

Die folgenden Beispiele und Vergleichsbeispiele dienen zur näheren Erläuterung der Erfindung.

In den Vergleichsbeispielen 1 und 2 konnte bei Abwandlung der in der DE-OS 32 33 764 beschriebenen Vorgehensweise kein positives Ergebnis im Sinne einer Stabilität der Arzneiform gegenüber dem Einfluss von simuliertem Magensaft mit pH 5 erreicht werden.

Andererseits erwiesen sich Placebo-Pellets, die bei pH 5 als stabil im Sinne von nicht-zerfallend, so dass die Vermutung nahe liegt, dass der Grund für die Instabilität mit dem alkalisch reagierenden Inhaltsstoff der Pellets zusammenhängt (Vergleichsbeispiel 3).

Überraschend wurde dann gefunden, dass eine magensaftresistente Umhüllung bei pH 5 dann erreichbar ist, wenn auf das wirkstoffhaltige Pellet ein Vorlack, bestehend aus HP[(R)]55 und - im Gegensatz zu DE-OS 32 33 764 - einer wasserunlöslichen organischen Säure, und ein Hauptlack, der im wesentlichen aus HP[(R)]50 besteht, aufgebracht wird. Derartig umhüllte Pellets sind bei pH 5 stabil und setzen unter den simulierten Bedingungen des oberen Dünndarms den Wirkstoff innerhalb von 20 Minuten vollständig frei.

Die Beispiele 1 und 2 verdeutlichen eine Herstellung der erfindungsgemässen Arzneiformen unter Einbezug derart magensaftresistent umhüllter Pellets und der gemäss Ausführungsbeispiel für Diclofenac-Natrium retardiert-durchlässig umhüllten Pellets.

Gemäss dem Beispiel 2 hergestellte Arzneiformen wurden in einer Pharmakokinetikstudie mit 12 Probanden geprüft. Die Versuchsanordnung entsprach dem "cross-over-design".

Frühere pharmakokinetischen Studien, in denen u.a. die Retardpellets, die auch für die erfindungsgemässe Kombination verwendet werden sollten, untersucht wurden, ergaben für diese Retardpellets in HGHK eine Zeitdauer zum Erreichen von Medianen > 100 ng/ml von > 2 h ab dem Einnahmezeitpunkt.

Aus der beiliegenden Fig. wird der der Erfindung zugrundeliegende Effekt deutlich. Die Arzneiform stellt einerseits ein rasches Erreichen von Plasmaspiegeln, die für eine therapeutische Wirkung erforderlich sind, sicher, andererseits ist die Dauer, während der die Plasmaspiegel die für eine Wirkung erforderliche minimale Plasmakonzentration überschreiten, derjenigen einer konventionellen Retardarzneiform vergleichbar.

Die in der vorliegenden Beschreibung, insbesondere den Beispielen und Vergleichsbeispielen verwendeten Handelsnamen haben folgende chemische Bedeutung:

Eudragit[(R)] RL          Polymerisat aus Acryl- und Methacrylester mit einem geringen Gehalt an quaternären Ammoniumgruppen für leicht durchlässige retardierende Filmüberzüge, (10% Trimethylammoniummethacrylatchlorid);

Eudragit(R) RS    wie vorstehend, (5% Trimethylammoniummethacrylatchlorid) jedoch für schwerdurchlässige retardierende Filmüberzüge;

Eudragit(R)L    anionisches Polymerisat aus Methacrylsäure und Methacrylsäuremethylester für magensaftresistente Filmüberzüge, darmsaftlöslich ab pH 6;

Eudragit(R)L 30 D    anionisches Copolymerisat auf Basis von Methacrylsäure und Acrylsäureethylester für magensaftresistente Filmüberzüge, darmsaftlöslich ab pH 5,5;

Eudragit(R)S    anionisches Polymerisat aus Methacrylsäure und Methacrylsäuremethylester für magensaftresistente Filmüberzüge, darmsaftlöslich ab pH 7.

Die Spezifikationen der vorstehend angegebenen Polymethylacrylate entsprechen denen, wie sie im Handbook of Pharmaceutical Excipients of The Pharmaceutical Society of Great Britain auf den Seiten 214 bis 217 angegeben sind.

HP-50    Hydroxypropylmethylcellulosephthalat mit einem durchschnittlichen Molekulargewicht von 20.000, und einem Methoxygruppenanteil von 20 bis 25%, einem Hydroxypropoxygruppenanteil von 5 bis 10% und einem Carboxybenzoylgruppenanteil von 20 bis 24%; Viskosität 240±48 mNs/m$^2$;

HP-55    Hydroxypropylmethylcellulosephthalat mit einem durchschnittlichen Molekulargewicht von 20.000, und einem Methoxygruppenanteil von 18 bis 22%, einem Hydroxypropoxygruppenanteil von 4 bis 9% und einem Carboxybenzoylgruppenanteil von 27 bis 35%; Viskosität 190±38 mNs/m$^2$.

HP-50 und HP-55 entsprechen den Spezifikationen, wie im Handbook of Pharmaceutical Excipients of The Pharmaceutical Society of Great Britain auf den Seiten 141 bis 144 angegeben.

Vergleichsbeispiele und Ausführungsbeispiele gemäss der Erfindung

Herstellung der wirkstoffhaltigen Pellets:

Gemäss bekannten Pelletisierverfahren wurden 20 kg wirkstoffhaltige Pellets mit folgender Zusammensetzung hergestellt:

| | |
|---|---|
| Diclofenac-Natrium | 40 % |
| Saccharose | 55 % |
| Poly(1-vinyl-2-pyrrolidon) | 4 % |
| Hochdisperses Siliciumdioxid | 1 % |

Vergleichsbeispiel 1

Jeweils 1 kg der wirkstoffhaltigen Pellets wurden in einer Wirbelschichtapparatur mit folgenden Lackrezepturen überzogen:

| Vorlack | |
|---|---|
| Hydroxypropylmethylcellulose 15 mPa.s | 30 g |
| Hydroxypropylmethylcellulose 5 mPa.s | 20 g |
| Citronensäure | 6 g |
| Ethanol | 450 g |
| Dichlormethan | 450 g |

| Hauptlack | | |
|---|---|---|
| | 3a | 3b |
| HP$^{(R)}$ 50 | 100 g | 200 g |
| 2-Propanol | 450 g | 900 g |
| Wasser | 450 g | 900 g |

In-vitro-Wirkstoff-Freisetzung (%) bzw. organoleptische Prüfung auf Aussehen:

| Zeit (Min.) | pH 1,2 | | pH 5,0 | |
|---|---|---|---|---|
| | 3a | 3b | 3a | 3b |
| 30 | | | Die Pellets sind zerfallen | |
| 120 | 8,0 | 6,1 | | |

### Vergleichsbeispiel 2

Jeweils 1 kg der wirkstoffhaltigen Pellets wurden in einer Wirbelschichtapparatur mit folgenden Lackrezepturen überzogen:

| Vorlack | |
|---|---|
| HP$^{(R)}$ 55 | 50 g |
| Citronensäure | 7 g |
| Aceton | 286 g |
| Ethanol | 286 g |

| Hauptlack | | |
|---|---|---|
| | 4a | 4b |
| HP$^{(R)}$ 50 | 100 g | 200 g |
| 2-Propanol | 450 g | 900 g |
| Wasser | 450 g | 900 g |

In-vitro-Wirkstoff-Freisetzung (%) bzw. organoleptische Prüfung auf Aussehen:

| Zeit (Min.) | pH 1,2 | | pH 5,0 | |
|---|---|---|---|---|
| | 4a | 4b | 4a | 4b |
| 30 | | | Die Pellets sind zerfallen | |
| 120 | 3,2 | 2,1 | | |

### Vergleichsbeispiel 3

Gemäss bekannten Pelletisierverfahren wurden 2 kg wirkstoff-freie Pellets mit folgenden Zusammensetzungen hergestellt:

| Saccharose | 78 % |
|---|---|
| Maisstärke | 15 % |
| Poly(1-vinyl-2-pyrrolidon) | 7 % |

Jeweils 1 kg der wirkstoff-freien Pellets wurden in einer Wirbelschichtapparatur mit folgenden Lackrezepturen überzogen:

| | 6a | 6b |
|---|---|---|
| HP$^{(R)}$ 50 | 100 g | 200 g |
| Aceton | 950 g | 1900 g |
| Ethanol | 950 g | 1900 g |

Organoleptische Prüfung auf Aussehen:

| Zeit (Min.) | pH 1,2 | | pH 5,0 | |
|---|---|---|---|---|
| | 6a | 6b | 6a | 6b |
| 30 | | | Kein Zerfall der Pellets | |
| 120 | kein Zerfall der Pellets | | | |

Beispiel 1

Ausführungsbeispiel zur magensaftresistenten Umhüllung der Pellets gemäss der Erfindung

Jeweils 1 kg der wirkstoffhaltigen Pellets wurden in einer Wirbelschichtapparatur mit folgenden Lackrezepturen überzogen:

| Vorlack | |
|---|---|
| HP$^{(R)}$55 | 50 g |
| Stearinsäure | 143 g |
| Talkum | 25 g |
| Aceton | 286 g |
| Ethanol | 286 g |

| Hauptlack | |
|---|---|
| HP$^{(R)}$50 | 100 g |
| Acetylierte Monoglyceride | 10 g |
| Talkum | 50 g |
| 2-Propanol | 450 g |
| Wasser | 450 g |

In-vitro-Wirkstoff-Freisetzung (%) bzw. organoleptische Prüfung auf Aussehen:

8

| Zeit (min.) | pH 1,2 | pH 5,0 | pH 6,8 |
|---|---|---|---|
| 5 | | | 5 |
| 10 | | | 74 |
| 20 | | | 103 |
| 30 | | kein Zerfall der Pellets | |
| 120* | 0 | | |

* Nach 120 Minuten wurde der pH von 1,2 auf 5,0 eingestellt; nach weiteren 30 Minuten erfolgt die Einstellung des pH-Wertes auf 6,8 (die sonstigen Bedingungen entsprachen dem Europäischen Arzneibuch).

Beispiel 2

Ausführungsbeispiel zur retardiert durchlässigen Umhüllung der Pellets gemäss der Erfindung

Jeweils 1 kg der wirkstoffhaltigen Pellets wurden in einer Wirbelschichtapparatur mit folgender Lackrezeptur überzogen:

| | |
|---|---|
| Eudragit[R] RS 100 | 20 g |
| Eudragit[R] RL 100 | 10 g |
| Talkum | 30 g |
| Phthalsäuredibutylester | 3 g |
| Aceton | 215 g |
| 2-Propanol | 322 g |

In-vitro-Wirkstoff-Freisetzung (%):

| Zeit (h) | pH 1,2 | pH 6,8 |
|---|---|---|
| 1 | <10 | |
| 2 | | 5 - 30 |
| 4 | | 50 - 85 |
| 6 | | > 70 |

Beispiel 3:

Ausführungsbeispiel zur Herstellung der erfindungsgemässen Arzneiformen

In Hartgelatinekapseln der Grösse 2 wurden jeweils 80,5 mg der magensaftresistent umhüllten Pellets und jeweils 130,5 mg der retardiert durchlässigen Pellets mit einer geeigneten Kapselabfüllmaschine eindosiert. Der Kapselinhalt hatte folgende Zusammensetzung:

| | Magensaftresistent umhüllte Pellets (mg) | retardiert durchlässige Pellets (mg) |
|---|---|---|
| Diclofenac-Natrium | 25,0 | 50,0 |
| Saccharose | 34,4 | 68,8 |
| Poly(1-vinyl-2-pyrrolidon) | 2,5 | 5,0 |
| Hochdisperses Siliciumdioxid | 0,6 | 1,3 |
| HP(R)55 | 2,5 | |
| HP(R)50 | 5,0 | |
| Stearinsäure | 7,2 | |
| Acetylierte Monoglyceride | 0,5 | |
| Talkum | 2,8 | |
| Eudragit(R) RS 100 | | 1,9 |
| Eudragit(R) RL 100 | | 0,7 |
| Talkum | | 2,5 |
| Phthalsäuredibutylester | | 0,3 |
| | 80,5 | 130,5 |

## Beispiel 4

Bei einer anderen bevorzugten Ausführungsform wurden in Hartgelatinekapseln der Grösse 1 jeweils 83,3 mg der magensaftresistent umhüllten Pellets und jeweils 196,8 mg der retardiert durchlässigen Pellets mit einer geeigneten Kapselabfüllmaschine eindosiert. Der Kapselinhalt hatte folgende Zusammensetzung:

| | Magensaftresistent umhüllte Pellets (mg) | retardiert durchlässige Pellets (mg) |
|---|---|---|
| Diclofenac-Natrium | 25,0 | 75,0 |
| Saccharose | 34,4 | 103,1 |
| Poly(1-vinyl-2-pyrrolidon) | 2,5 | 7,5 |
| Hochdisperses Siliciumdioxid | 0,6 | 1,9 |
| HP(R)55 | 2,5 | |
| HP(R)50 | 5,0 | |
| Stearinsäure | 7,2 | |
| Acetylierte Monoglyderide | 0,5 | |
| Talkum | 2,8 | |
| Eudragit(R) RS 100 | | 2,9 |
| Eudragit(R) RL 100 | | 1,0 |
| Talkum | 2,8 | |
| Eudragit(R) RS 100 | | 1,9 |
| Eudragit(R) RL 100 | | 0,7 |
| Talkum | | 2,5 |
| Phthalsäuredibutylester | | 0,3 |
| | 83,3 | 196,8 |

## Beispiel 5

Bei einer anderen bevorzugten Ausführungsform wurden in Hartgelatinekapseln der Grösse 1 jeweils 80,5 mg der magensaftresistent umhüllten Pellets und jeweils 195,8 mg der retardiert durchlässigen Pellets mit einer geeigneten Kapselabfüllmaschine eindosiert. Der Kapselinhalt hatte folgende Zusammensetzung:

| | Magensaftresistent umhüllte Pellets (mg) | retardiert durchlässige Pellets (mg) |
|---|---|---|
| Diclofenac-Natrium | 25,0 | 75,0 |
| Saccharose | 34,4 | 103,1 |
| Poly(1-vinyl-2-pyrrolidon) | 2,5 | 7,5 |
| Hochdisperses Siliciumdioxid | 0,6 | 1,9 |
| HP(R)55 | 2,5 | |
| HP(R)50 | 5,0 | |
| Stearinsäure | 7,2 | |
| Acetylierte Monoglyderide | 0,5 | |
| Talkum | 2,8 | |
| Eudragit(R) RS 100 | | 2,9 |
| Eudragit(R) RL 100 | | 1,0 |
| Talkum | | 3,9 |
| Phthalsäuredibutylester | | 0,5 |
| | 80,5 | 195,8 |

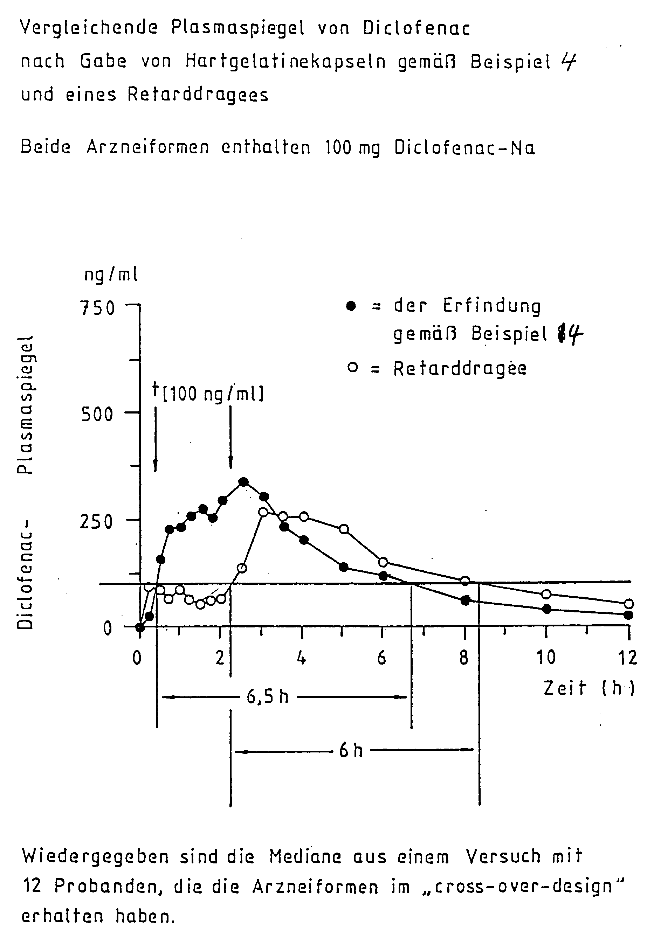

**Patentansprüche**

**Patentansprüche für folgende Vertragsstaaten : AT, BE, CH, LI, DE, FR, GB, IT, LU, NL, SE**

1. Diclofenac-Natrium enthaltendes Arzneimittel mit gesteuerter Wirkstoff-Freisetzung, dadurch **gekennzeichnet,** daß der Wirkstoff zu einem Teil in retardiert freisetzender und zum restlichen Teil in einer magensaftresistenten Form vorliegt, wobei der genannte Wirkstoff in oder auf sphärischen Pellets fein verteilt ist und ein Teil der Pellets mit einer für den Wirkstoff retardiert durchlässigen Diffusionsmembran und der restliche Teil der Pellets mit einer magensaftresistenten Membran umhüllt ist, und die genannten Pellets in einer Gelatinekapsel eingeschlossen sind.

2. Arzneimittel nach Anspruch 1, dadurch **gekennzeichnet,** daß die Kapsel eine Hartgelatinekapsel darstellt.

3. Arzneimittel nach Anspruch 1 und 2, dadurch **gekennzeichnet,** daß das Gewichtsverhältnis der Pellets mit einer magensaftresistenten Membran zu denen, die mit der Diffusionsmembran überzogen sind, 0,1:1 bis 2:1 beträgt.

4. Arzneimittel nach Anspruch 1 bis 3, dadurch **gekennzeichnet,** daß es in Kapseln abgefüllt ist, die jeweils 10 bis 500 mg Wirkstoff enthalten.

5. Arzneimittel nach Anspruch 1 bis 4, dadurch **gekennzeichnet,** daß sich die magensaftresistente Membran aus zwei Schichten Celluloseether mit unterschiedlichem Substitutionsgrad zusammensetzt, die mit Phthalsäureanhydrid verestert sind, und die innere Schicht als weiteren Hilfsstoff eine wasserunlösliche organische Säure sowie gegebenenfalls weitere Hilfsstoffe umfaßt.

6. Arzneimittel nach Anspruch 1 bis 5, dadurch **gekennzeichnet,** daß die retardiert durchlässige Diffusionsmembran aus Acrylharz besteht.

7. Arzneimittel nach einem oder mehreren der vorangehenden Ansprüche, dadurch **gekennzeichnet,** daß sich die magensaftresistente Membran zusammensetzt aus einem Vorlack, welcher Hydroxypropylmethylcellulosephthalat und eine in Wasser unlösliche organische Säure umfaßt, und einem Hauptlack, welcher Hydroxypropylmethylcellulosephthalat umfaßt, und die retardiert durchlässige Diffusionsmembran ein oder mehrere Acrylharzschichten umfaßt, wobei das Arzneimittel mit der genannten Membran in einer Kapsel eingeschlossen ist.

8. Arzneimittel nach Anspruch 7, dadurch **gekennzeichnet,** daß die organische Säure Stearinsäure darstellt.

**9.** Arzneimittel nach Anspruch 7, dadurch **gekennzeichnet,** daß sich die Acrylharzschicht aus einer schwer durchlässigen inneren und einer leicht durchlässigen äußeren Schicht zusammensetzt.

**10.** Arzneimittel nach Anspruch 7, dadurch **gekennzeichnet,** daß das Acrylharz ein Polymerisat aus Acryl- und Methacrylsäureester mit einem geringen Gehalt an quaternären Ammoniumgruppen darstellt.

**11.** Arzneimittel nach einem oder mehreren der vorangehenden Ansprüche dadurch **gekennzeichnet,** daß sich die zweischichtige magensaftresistente Membran, bezogen auf jeweils 1 kg Pellets, wie folgt zusammensetzt:

| Vorlack: | |
|---|---|
| Hydroxypropylmethylcellulosephthalat mit einem durchschnittlichen Molekulargewicht von 20.000, und einem Methoxygruppenanteil von 18 bis 22%, einem Hydroxypropoxygruppenanteil von 4 bis 9% und einem Carboxybenzoylgruppenanteil von 27 bis 35%; Vis kosität $190\pm38$ mNs/m$^2$. | 50 g |
| Stearinsäure | 143 g |
| Talkum | 25 g |

| Hauptlack: | |
|---|---|
| Hydroxypropylmethylcellulosephthalat mit einem durchschnittlichen Molekulargewicht von 20.000, und einem Methoxygruppenanteil von 20 bis 25%, einem Hydroxypropoxygruppenanteil von 5 bis 10% und einem Carboxybenzoylgruppenanteil von 20 bis 24%; Vi skosität $240\pm48$ mNs/m$^2$; | 100 g |
| Acetylierte Monoglyceride | 10 g |

und die retardiert durchlässige Membran zweischichtig ist und, jeweils pro 1 kg Pellets, folgende Zusammensetzung umfaßt:

| Polymerisat aus Acryl- und Methacrylester mit einem geringen Gehalt an quaternären Ammoniumgruppen für leicht durchlässige retardierende Filmüberzüge, (10% Trimethylammoniummethacrylatchlorid); | 10 g |
|---|---|
| wie vorstehend, (5% Trimethylammoniummethacrylatchlorid) jedoch für schwerdurchlässige retardierende Filmüberzüge; | 20 g |
| Talkum | 30 g |
| Phthalsäuredibutylester | 3 g |

**12.** Arzneimittel nach einem oder mehreren der Ansprüche 1 bis 4, dadurch **gekennzeichnet,** daß zur Herstellung der magensaftresistenten Pellets jeweils 62,5 g wirkstoffhaltige Pellets mit einer magensaftresistenten Membran überzogen werden, die 7,5 g Polymere umfaßt.

**13.** Arzneimittel nach einem oder mehreren der Ansprüche 1 bis 4, dadurch **gekennzeichnet,** daß zur Herstellung der magensaftresistenten Pellets jeweils 1 kg wirkstoffhaltige Pellets mit einer magensaftresistenten Membran überzogen werden, die 150 g Polymere umfaßt.

**Patentansprüche für folgende Vertragsstaaten : ES, GR**

**1.** Verfahren zur Herstellung eines Diclofenac-Natrium enthaltenden Arzneimittels mit gesteuerter Wirkstoff-Freisetzung, dadurch gekennzeichnet, daß der Wirkstoff zu einem Teil in retardiert freisetzender und zum restlichen Teil in einer magensaftresistenten Form formuliert wird, wobei der genannte Wirkstoff in oder auf sphärischen Pellets fein verteilt wird und ein Teil der Pellets mit einer für den Wirkstoff retardiert durchlässigen Diffusionsmembran und der restliche Teil der Pellets mit einer magensaftresistenten Membran umhüllt wird, und die genannten Pellets in einer Gelatinekapsel eingeschlossen werden.

2. Verfahren nach Anspruch 1, dadurch gekennzeichnet, daß als Kapsel eine Hartgelatinekapsel verwendet wird.

3. Verfahren nach Anspruch 1 und 2, dadurch gekennzeichnet, daß das Gewichtsverhältnis der Pellets mit einer magensaftresistenten Membran zu denen, die mit der Diffusionsmembran überzogen sind, auf 0.1:1 bis 2:1 eingestellt wird.

4. Verfahren nach Anspruch 1 bis 3, dadurch gekennzeichnet, daß das Arzneimittel in Kapseln abgefüllt wird, welche jeweils 10 bis 500 mg Wirkstoff enthalten.

5. Verfahren nach Anspruch 1 bis 4, dadurch gekennzeichnet, daß sich die magensaftresistente Membran aus zwei Schichten Celluloseether mit unterschiedlichem Substitutionsgrad zusammensetzt, die mit Schicht als weiteren Hilfstoff eine wasserunlösliche organische Säure sowie gegebenenfalls weitere Hilfstoffe umfaßt.

6. Verfahren nach Anspruch 1 bis 5, dadurch gekennzeichnet, daß die retardiert durchlässige Diffusionsmembran aus Acrylharz besteht.

7. Verfahren nach einem odere mehreren der vorangehenden Ansprüche, dadurch gekennzeichnet, daß sich die magensaftresistente Membran zusammensetzt aus einem Vorlack, welcher Hydroxypropylmethylcellulosephthalat und eine in Wasser unlösliche organische Säure umfaßt, und einem Hauptlack, welcher Hydroxypropylmethylcellulosephthalat umfaßt, und die retardiert durchlässige Diffusionsmembran ein oder mehrere Acrylharzschichten umfaßt, und das Arzneimittel mit der genannten Membran in einer Kapsel eingeschlossen wird.

8. Verfahren nach Anspruch 7, dadurch gekennzeichnet, daß als organische Säure Stearinsäure verwendet wird.

9. Verfahren nach Anspruch 7, dadurch gekennzeichnet, daß sich die Acrylharzschicht aus einer schwer durchlässigen inneren und einer leicht durchlässigen äußeren Schicht zusammensetzt.

10. Verfahren nach Anspruch 7, dadurch gekennzeichnet, daß als Acrylharz ein Polymerisat aus Acryl- und Methacrylsäureester mit einem geringen Gehalt an quaternären Ammoniumgruppen verwendet wird.

11. Verfahren nach einem oder mehreren der vorangehenden Ansprüche, dadurch gekennzeichnet, daß die zweischichtige magensaftresistente Membran, bezogen auf jeweils 1 kg Pellets, aus folgenden Kompo-nenten hergestellt wird:

| | |
|---|---|
| Vorlack:<br>Hydroxypropylmethylcellulose-<br>phthalat mit einem<br>durchschnittlichen<br>Molekulargewicht von 20.000, und<br>einem Methoxygruppenanteil von<br>18 bis 22 %, einem<br>Hydroxypropoxygruppenanteil von<br>4 bis 9 % und einem<br>Carboxybenzoylgruppenanteil von<br>27 bis 35 %;<br>Viskosität 190 ± 38 mNs/m² | 50 g |
| Stearinsäure | 143 g |
| Talkum | 25 G |
| Hauptlack:<br>Hydroxypropylmethylcellulose-<br>phthalat mit einem<br>durchschnittlichen<br>Molekulargewicht von 20.000, und<br>einem Methoxygruppenanteil von<br>20 bis 25 %, einem<br>Hydroxypropoxygruppenanteil von<br>5 bis 10 % und einem<br>Carboxybenzoylgruppenanteil von<br>20 bis 24 %;<br>Viskosität 240 ± 48 mNs/m²; | 100 g |
| Acetylierte Monoglyceride | 10 g |

und die retardiert durchlässige Membran zweischichtig ist und, jeweils pro 1 kg Pellets, aus folgenden Komponenten formuliert wird:

14

EP 0 348 808 B1

| | |
|---|---|
| Polymerisat aus Acryl- und Methacrylester mit einem geringen Gehalt an quaternären Ammoniumgruppen für leicht durchlässige retardierende Filmüberzüge, (10 % Trimethylammoniummethacrylatchlorid); | 10 g |
| wie vorstehend, (5 % Trimethylammoniummethacrylatchlorid) jedoch für schwerdurchlässige retardierende Filmüberzüge; | 20 g |
| Talkum | 30 g |
| Phthalsäuredibutylester | 3 g |

12. Verfahren nach einem oder mehreren der Ansprüche 1 bis 4, dadurch gekennzeichnet, daß für die Herstellung der magensaftresistenten Pellets jeweils 62.5 g wirkstoffhaltige Pellets mit einer magensaftresistenten Membran überzogen werden, die 7,5 g Polymere umfaßt.

13. Verfahren nach einem oder mehreren der Ansprüche 1 bis 4, dadurch gekennzeichnet, daß für die Herstellung der magensaftresistenten Pellets jeweils 1 kg wirkstoffhaltige Pellets mit einer magensaftresistenten Membran überzogen werden, die 150 g Polymere umfaßt.

**Claims**
**Claims for the following Contracting States : AT, BE, CH, LI, DE, FR, GB, IT, LU, NL, SE**

1. Medicament containing diclofenac sodium having controlled release of active ingredient, characterised in that the active ingredient exists partly in a delayed-releasing form and the remaining part exists in a gastric juice-resistant form, the active ingredient mentioned being finely distributed in or on spherical pellets and a part of the pellets being coated with a diffusion membrane which is permeable to the active ingredient in a delayed manner and the remaining part of the pellets being coated with a gastric juice-resistant membrane, and the pellets mentioned are enclosed in a gelatin capsule.

2. Medicament according to claim 1, characterised in that the capsule is a hard gelatin capsule.

3. Medicament according to claim 1 and 2, characterised in that the weight ratio of the pellets having a gastric juice-resistant membrane to those which are coated with the diffusion membrane is 0.1:1 to 2:1.

4. Medicament according to claim 1 to 3, characterised in that it is poured into capsules each containing 10 to 500 mg of active ingredient.

15

**5.** Medicament according to claim 1 to 4, characterised in that the gastric juice-resistant membrane is composed of two layers of cellulose ether having different degrees of substitution and which are esterified using phthalic acid anhydride, and the inner layer includes a water-insoluble organic acid as a further auxiliary as well as optional further auxiliaries.

**6.** Medicament according to claim 1 to 5, characterised in that the diffusion membrane which is permeable in a delayed manner consists of acrylic resin.

**7.** Medicament according to one or more of the preceding claims, characterised in that the gastric juice-resistant membrane is composed of an initial lacquer, which comprises hydroxypropylmethylcellulose phthalate and an organic acid which is insoluble in water, and a main lacquer, which comprises hydroxypropylmethylcellulose phthalate, and the diffusion membrane which is permeable in a delayed manner comprises one or more acrylic resin layers, the medicament being enclosed in a capsule by the membrane mentioned.

**8.** Medicament according to claim 7, characterised in that the organic acid is stearic acid.

**9.** Medicament according to claim 7, characterised in that the acrylic resin layer is composed of an inner layer which has poor permeability and an outer layer which has high permeability.

**10.** Medicament according to claim 7, characterised in that the acrylic resin is a polymer of acrylate and methacrylate with a low content of quaternary ammonium groups.

**11.** Medicament according to one or more of the preceding claims, characterised in that the two-layer gastric juice-resistant membrane, based in each case on 1 kg of pellets, has the following composition:

| Initial lacquer: | |
|---|---|
| Hydroxypropylmethylcellulose phthalate having an average molecular weight of 20,000, and a methoxy group portion of 18 to 22 %, a hydroxypropoxy group portion of 4 to 9 % and a carboxybenzoyl group portion of 27 to 35 %; viscosity 190 ± 38 mNs/m$^2$ . | 50 g |
| Stearic acid | 143 g |
| Talcum | 25 g |

| Main lacquer: | |
|---|---|
| Hydroxypropylmethylcellulose phthalate having an average molecular weight of 20,000, and a methoxy group portion of 20 to 25 %, a hydroxypropoxy group portion of 5 to 10 % and a carboxybenzoyl group portion of 20 to 24 %; viscosity 240 ± 48 mNs/m $^2$; | 100 g |
| Acetylated monoglycerides | 10 g |

and the membrane which is permeable in a delayed manner has two layers and, in each case per 1 kg of pellets, has the following composition:

| Polymer of acrylate and methacrylate with a low content of quaternary ammonium groups for film coatings which delay with high permeability, 10 % trimethylammonium methacrylate chloride); | 10 g |
|---|---|
| as above, but (5 % trimethylammonium methacrylate chloride) for film coatings which delay with low permeability; | 20 g |
| Talcum | 30 g |
| Dibutyl phthalate | 3 g |

**12.** Medicament according to one or more of claims 1 to 4, characterised in that in each case 62.5 g of pellets containing active ingredient are coated with a gastric juice-resistant membrane comprising 7.5 g of polymers to produce the gastric juice-resistant pellets.

13. Medicament according to one or more of claims 1 to 4, characterised in that in each case 1 kg of pellets containing active ingredient are coated with a gastric juice-resistant membrane comprising 150 g of polymers to produce the gastric juice-resistant pellets.

**Claims for the following Contracting States : ES, GR**

1. Process for producing a medicament containing diclofenac sodium having controlled release of active ingredient, characterised in that the active ingredient is formulated partly in a delayed-releasing form and the remaining part is formulated in a gastric juice-resistant form, the active ingredient mentioned being finely distributed in or on spherical pellets and a part of the pellets being coated with a diffusion membrane which is permeable to the active ingredient in a delayed manner and the remaining part of the pellets being coated with a gastric juice-resistant membrane, and the pellets mentioned are enclosed in a gelatin capsule.

2. Process according to claim 1, characterised in that a hard gelatin capsule is used as the capsule.

3. Process according to claim 1 and 2, characterised in that the weight ratio of the pellets having a gastric juice-resistant membrane to those which are coated with the diffusion membrane is adjusted to 0.1:1 to 2:1.

4. Process according to claim 1 to 3, characterised in that the medicament is poured into capsules each containing 10 to 500 mg of active ingredient.

5. Process according to claim 1 to 4, characterised in that the gastric juice-resistant membrane is composed of two layers of cellulose ether having different degrees of substitution and which are esterified using phthalic acid anhydride, and the inner layer includes a water-insoluble organic acid as a further auxiliary as well as optional further auxiliaries.

6. Process according to claim 1 to 5, characterised in that the diffusion membrane which is permeable in a delayed manner consists of acrylic resin.

7. Process according to one or more of the preceding claims, characterised in that the gastric juice-resistant membrane is composed of an initial lacquer, which comprises hydroxypropylmethylcellulose phthalate and an organic acid which is insoluble in water, and a main lacquer, which comprises hydroxypropylmethylcellulose phthalate, and the diffusion membrane which is permeable in a delayed manner comprises one or more acrylic resin layers, and the medicament is enclosed in a capsule by the membrane mentioned.

8. Process according to claim 7, characterised in that stearic acid is used as the organic acid.

9. Process according to claim 7, characterised in that the acrylic resin layer is composed of an inner layer which has poor permeability and an outer layer which has high permeability.

10. Process according to claim 7, characterised in that a polymer of acrylate and methacrylate with a low content of quaternary ammonium groups is used as acrylic resin.

11. Process according to one or more of the preceding claims, characterised in that the two-layer gastric juice-resistant membrane, based in each case on 1 kg of pellets, is produced from the following components:

17

```
Initial lacquer:

Hydroxypropylmethylcellulose phthalate having
an average molecular weight of 20,000, and a
methoxy group portion of 18 to 22 %, a
hydroxypropoxy group portion of 4 to 9 % and a
carboxybenzoyl group portion of 27 to 35 %;
viscosity 190 ± 38 mNs/m²                              50 g


Stearic acid                                          143 g
Talcum                                                 25 g

Main lacquer:

Hydroxypropylmethylcellulose phthalate having
an average molecular weight of 20,000, and a
methoxy group portion of 20 to 25 %, a
hydroxypropoxy group portion of 5 to 10 % and
a carboxybenzoyl group portion of 20 to 24 %;
viscosity 240 ± 48 mNs/m²;                            100 g

Acetylated monoglycerides                              10 g
```

and the membrane which is permeable in a delayed manner has two layers and, in each case per 1 kg of pellets, is formulated from the following components:

```
Polymer of acrylate and methacrylate with a
low content of quaternary ammonium groups for
film coatings which delay with high permeability,
10 % trimethylammonium methacrylate
chloride);                                            10 g

as above, but (5 % trimethylammonium methacrylate
chloride) for film coatings which delay
with low permeability;                                20 g

Talcum                                               30 g
Dibutyl phthalate                                     3 g
```

12. Process according to one or more of claims 1 to 4, characterised in that in each case 62.5 g of pellets containing active ingredient are coated with a gastric juice-resistant membrane comprising 7.5 g of polymers to produce the gastric juice-resistant pellets.

13. Process according to one or more of claims 1 to 4, characterised in that in each case 1 kg of pellets containing active ingredient are coated with a gastric juice-resistant membrane comprising 150 g of polymers to produce the gastric juice-resistant pellets.

18

**EP 0 348 808 B1**

**Revendications**
**Revendications pour les Etats contractants suivants : AT, BE, CH, LI, DE, FR, GB, IT, LU, NL, SE**

1. Médicament contenant du diclofénacsodium à libération contrôlée de la substance active, caractérisé en ce que la substance active est présente pour une part sous une forme à libération retardée et pour le reste sous une forme résistant au suc gastrique, cette substance active étant finement répartie dans ou sur des granulés sphériques et une partie des granulés étant enrobée d'une membrane de diffusion perméable avec retard pour la substance active et la partie restante des granulés étant enrobée d'une membrane résistant au suc gastrique, et ces granulés étant inclus dans une capsule de gélatine.

2. Médicament selon les revendications 1, caractérisé en ce que la capsule est une capsule de gélatine dure.

3. Médicament selon les revendications 1 et 2, caractérisé en ce que le rapport pondéral des granulés dont la membrane résiste au suc gastrique à ceux qui sont revêtus de la membrane de diffusion est de 0,1 : 1 à 2 : 1.

4. Médicament selon les revendications 1 à 3, caractérisé en ce qu'il est introduit dans des capsules qui contiennent à chaque fois 10 à 500 mg de substance active.

5. Médicament selon les revendications 1 à 4, caractérisé en ce que la membrane résistant au suc gastrique se compose de deux couches d'éther de cellulose ayant des degrés de substitution différents, qui sont estérifiées avec de l'anhydride phtalique, et en ce que la couche interne comprend comme autre substance auxiliaire un acide organique insoluble dans l'eau ainsi que le cas échéant d'autres substances auxiliaires.

6. Médicament selon les revendications 1 à 5, caractérisé en ce que la membrane de diffusion perméable avec retard se compose d'une résine acrylique.

7. Médicament selon une ou plusieurs des revendications précédentes, caractérisé en ce que la membrane résistant au suc gastrique se compose d'une sous-couche qui comprend du phtalate d'hydroxypropylméthylcellulose et un acide organique insoluble dans l'eau, et d'une couche principale qui comprend du phtalate d'hydroxypropylméthylcellulose, et en ce que la membrane de diffusion perméable avec retard comprend une ou plusieurs couches de résine acrylique, le médicament portant la membrane indiquée étant inclus dans une capsule.

8. Médicament selon la revendication 7, caractérisé en ce que l'acide organique est l'acide stéarique.

9. Médicament selon la revendication 7, caractérisé en ce que la couche de résine acrylique se compose d'une couche interne peu perméable et d'une couche externe très perméable.

10. Médicament selon la revendication 7, caractérisé en ce que la résine acrylique est un polymère d'un ester acrylique et méthacrylique ayant une faible teneur en groupes ammonium quaternaire.

11. Médicament selon une ou plusieurs des revendications précédentes, caractérisé en ce que la membrane à deux couches résistant au suc gastrique présente la composition suivante, rapportée à chaque fois à 1 kg de granulés :

| Sous-couche : | |
|---|---|
| Phtalate d'hydroxypropylméthylcellulose ayant une masse moléculaire moyenne de 20 000 et une teneur en groupes méthoxy de 18 à 22 %, une teneur en groupes hydroxypropoxy de 4 à 9 %, et une teneur en groupes carboxybenzoyle de 27 à 35 %; viscosité 190±38 mNs/m$^2$ | 50 g |
| Acide stéarique | 143 g |
| Talc | 25 g |

| Couche principale : | |
|---|---|
| Phtalate d'hydroxypropylméthylcellulose ayant une masse moléculaire moyenne de 20 000 et une proportion de groupes méthoxy de 20 à 25 %, une proportion de groupes hydroxypropoxy de 5 à 10 % et une proportion de groupes carboxybenzoyle de 20 à 24 %; Viscosité 240±48 mNs/m$^2$; | 100 g |
| Monoglycérides acétylés | 10 g |

et en ce que la membrane perméable avec retard est formée de deux couches et comprend la composition suivante, rapportée à chaque fois à 1 kg de granulés :

| Polymère d'ester acrylique et méthacrylique ayant une faible teneur en groupes ammonium quaternaire pour enrobages de film retardateurs très perméables (10 % de chlorure du méthacrylate de triméthylammonium); | 10 g |
|---|---|
| comme précédemment, (5 % de chlorure du méthacrylate de triméthylammonium), mais pour des enrobages retardateurs peu perméables; | 20 g |
| Talc | 30 g |
| Phtalate de dibutyle | 3 g |

**12.** Médicament selon une ou plusieurs des revendications 1 à 4, caractérisé en ce que pour la préparation des granulés résistant au suc gastrique, on revêt à chaque fois 62,5 g de granulés contenant la substance active d'une membrane résistant au suc gastrique qui comprend 7,5 g de polymères.

**13.** Médicament selon une ou plusieurs des revendications 1 à 4, caractérisé en ce que pour la préparation des granulés résistant au suc gastrique, on revêt à chaque fois 1 kg de granulés contenant la substance active d'une membrane résistant au suc gastrique qui comprend 150 g de polymères.

**Revendications pour les Etats contractants suivants : ES, GR**

**1.** Procédé pour la préparation d'un médicament contenant du diclofénac-sodium à libération contrôlée de la substance active, caractérisé en ce que la substance active est formulée pour une part sous une forme à libération retardée et pour le reste sous une forme résistant au suc gastrique, cette substance active étant finement répartie dans ou sur des granulés sphériques et une partie des granulés étant enveloppée dans une membrane de diffusion perméable avec retard à la substance active et l'autre partie des granulés étant enveloppée dans une membrane résistant au suc gastrique, et en ce que ces granulés sont inclus dans une capsule de gélatine.

**2.** Procédé selon la revendication 1, caractérisé en ce qu'on utilise comme capsule une capsule de gélatine dure.

**3.** Procédé selon selon les revendications 1 et 2, caractérisé en ce que le rapport pondéral des granulés ayant une membrane résistant au suc gastrique à ceux qui sont revêtus d'une membrane de diffusion est ajusté à 0,1 : 1 à 2 : 1.

**4.** Procédé selon les revendications 1 à 3, caractérisé en ce que le médicament est introduit dans dès capsules qui contiennent à chaque fois 10 à 500 mg de substance active.

**5.** Procédé selon les revendications 1 à 4, caractérisé en ce que la membrane résistant au suc gastrique se compose de deux couches d'éther de cellulose de degrés de substitution différents, qui sont estérifiées par l'anhydride phtalique, et en ce que la couche interne comprend comme autre substance auxiliaire un acide organique insoluble dans l'eau et le cas échéant d'autres substances auxiliaires.

**6.** Procédé selon les revendications 1 à 5, caractérisé en ce que la membrane de diffusion perméable avec retard se compose d'une résine acrylique.

7. Procédé selon une ou plusieurs des revendications précédentes, caractérisé en ce que la membrane résistant au suc gastrique se compose d'une sous-couche qui comprend du phtalate d'hydroxypropylméthylcellulose et un acide organique insoluble dans l'eau, et d'une couche principale qui comprend du phtalate d'hydroxypropylméthylcellulose, et en ce que la membrane perméable avec retard comprend une ou plusieurs couches de résine acrylique, et en ce que le médicament portant la membrane indiquée est inclus dans une capsule.

8. Procédé selon la revendication 7, caractérisé en ce que l'on utilise comme acide organique de l'acide stéarique.

9. Procédé selon la revendication 7, caractérisé en ce que la couche de résine acrylique se compose d'une couche interne peu perméable et d'une couche externe très perméable.

10. Procédé selon la revendication 7, caractérisé en ce qu'on utilise comme résine acrylique un polymère d'un ester acrylique et méthacrylique ayant une faible teneur en groupes ammonium quaternaire.

11. Procédé selon une ou plusieurs des revendications précédentes, caractérisé en ce que la membrane à deux couches résistant au suc gastrique est préparée, pour 1 kg à chaque fois de granulés, à partir des constituants suivants:

| | |
|---|---|
| Sous-couche :<br>Phtalate d'hydroxypropylméthylcellulose ayant une masse moléculaire moyenne de 20 000 et une proportion de groupes méthoxy de 18 à 22 %, une proportion de groupes hydroxypropoxy de 4 à 9 %, et une proportion de groupes carboxy-benzoyle de 27 à 35 %;<br>Viscosité $190 \pm 38$ mNs/m$^2$ | 50 g |
| Acide stéarique | 143 g |
| Talc | 25 g |
| Couche principale :<br>Phtalate d'hydroxypropylméthylcellulose ayant une masse moléculaire moyenne de 20 000, une proportion de groupes méthoxy de 20 à 25 %, une proportion de groupes hydroxypropoxy de 5 à 10 % et une proportion de groupes carboxybenzoyle de 20 à 24 %;<br>Viscosité $240 \pm 48$ mNs/m$^2$; | 100 g |
| Monoglycérides acétylés | 10 g |

et en ce que la membrane perméable avec retard est composée de deux couches et est formulée, pour 1 kg de granulés à chaque fois, à partir des constituants suivants :

| | |
|---|---|
| Polymère d'ester acrylique et méthacrylique ayant une faible teneur en groupes ammonium quaternaire pour des enrobages retardateurs très perméables (10 % de chlorure du méthacrylate de triméthylammonium); comme précédemment, (5 % de chlorure du méthacrylate de triméthylammonium), mais pour des enrobages de films retardateurs peu perméables; | 10 g

20 g |
| Talc | 30 g |
| Phtalate de dibutyle | 3 g |

12. Procédé selon une ou plusieurs des revendications 1 à 4, caractérisé en ce que pour la préparation des granulés résistant au suc gastrique, on revêt à chaque fois 62,5 g de granulés contenant la substance active d'une membrane résistant au suc gastrique, qui contient 7,5 g de polymères.

13. Procédé selon une ou plusieurs des revendications 1 à 4, caractérisé en ce que pour la préparation des granulés résistant au suc gastrique, on revêt à chaque fois 1 kg de granulés contenant la substance active d'une membrane résistant au suc gastrique qui contient 150 g de polymères.

EP 0 348 808 B1

Vergleichende Plasmaspiegel von Diclofenac
nach Gabe von Hartgelatinekapseln gemäß Beispiel 4
und eines Retarddragees

Beide Arzneiformen enthalten 100 mg Diclofenac-Na

Wiedergegeben sind die Mediane aus einem Versuch mit
12 Probanden, die die Arzneiformen im „cross-over-design"
erhalten haben.

23